# EUROPEAN PATENT SPECIFICATION

(11) **EP 2 497 494 B1**
(45) Date of publication and mention of the grant of the patent: **24.01.2018**
(21) Application number: 10809153.9
(22) Date of filing: 05.11.2010
(51) Int. Cl.: A61K 39/12

(54) **VACCINES FOR THE TREATMENT OF NEOPLASIAS FROM VIRAL CAPSIDS OF BIRNAVIRUS CONTAINING ANTIGENS OF THE HUMAN PAPILLOMAVIRUS**
IMFPSTOFFE ZUR BEHANDLUNG VON NEOPLASIEN AUS VIRALEN BIRNAVIRUS-KAPSIDEN MIT ANTIGENEN DES HUMANEN PAPILLOMAVIRUS
VACCINS POUR LE TRAITEMENT DE NÉOPLASIES À PARTIR DE CAPSIDES VIRALES DE BIRNAVIRUS CONTENANT DES ANTIGÈNES DU VIRUS DU PAPILLOME HUMAIN

(30) Priority: 06.11.2009 ES 200930967
(43) Date of publication of application: 12.09.2012
(73) Proprietor: Chimera Pharma, S. L. U., 28760 Tres Cantos (Madrid) (ES)
(72) Inventor: ZURCHER, Thomas, E-28760 Tres Cantos (Madrid) (ES); VON KOBBE, Cayetano, E-28760 Tres Cantos (Madrid) (ES); BERNAL, Juan José, E-28760 Tres Cantos (Madrid) (ES); JIMENEZ TORRES, Ignacio, E-28760 Tres Cantos (Madrid) (ES); CALDERITA LUCAS, Gloria, E-28760 Tres Cantos (Madrid) (ES); RODRIGUEZ GARCIA, Margarita, E-28760 Tres Cantos (Madrid) (ES); GARZON GUTIERREZ, Ana, E-28760 Tres Cantos (Madrid) (ES); GONDAR SOUSA E SILVA, Virginia, E-28760 Tres Cantos (Madrid) (ES); GARCIA DE CASTRO, Arcadio, E-28760 Tres Cantos (Madrid) (ES); PINO DE LA HUERGA, Irene, E-28760 Tres Cantos (Madrid) (ES)
(74) Representative: Pons
(86) International application number: PCT/ES2010/070717
(87) International publication number: WO 2011/054996

(56) References cited:
- EP-A1- 2 093 281
- US-A1- 2007 015 243
- US-A1- 2007 196 339
- TINDLE R W ET AL: "CHIMERIC HEPATITIS B CORE ANTIGEN PARTICLES CONTAINING B- AND TH-EPITOPES OF HUMAN PAPILLOMAVIRUS TYPE 16 E7 PROTEIN INDUCE SPECIFIC ANTIBODY AND T-HELPER RESPONSES IN IMMUNISED MICE", VIROLOGY, ACADEMIC PRESS,ORLANDO, US, vol. 200, no. 2, 1 May 1994 (1994-05-01), pages 547-557, XP002035981, ISSN: 0042-6822, DOI: DOI:10.1006/VIRO.1994.1217 cited in the application
- GARRIGA DAMIA ET AL: "The 2.6-angstrom structure of infectious Bursal disease virus-derived T=1 particles reveals new stabilizing elements of the virus capsid", JOURNAL OF VIROLOGY, vol. 80, no. 14, July 2006 (2006-07), pages 6895-6905, XP002639160, ISSN: 0022-538X
- REMOND M ET AL: "Infectious bursal disease subviral particles displaying the foot-and-mouth disease virus major antigenic site", VACCINE, ELSEVIER LTD, GB, vol. 27, no. 1, 1 January 2009 (2009-01-01), pages 93-98, XP025714114, ISSN: 0264-410X, DOI: DOI:10.1016/J.VACCINE.2008.10.036 [retrieved on 2008-11-05] cited in the application
- WINTERS URSULA ET AL: "Progress in the development of a cervical cancer vaccine.", THERAPEUTICS AND CLINICAL RISK MANAGEMENT SEP 2006 LNKD- PUBMED:18360601, vol. 2, no. 3, September 2006 (2006-09), pages 259-269, XP002639161, ISSN: 1176-6336
- YOUSUF SADAF ET AL: "Prophylactic and therapeutic human papillomavirus vaccine: a breakthrough for women health", PAKISTAN MEDICAL ASSOCIATION. JOURNAL, PAKISTAN MEDICAL ASSOCIATION, KARACHI, PK, vol. 59, no. 5, 1 May 2009 (2009-05-01), pages 310-314, XP009148802, ISSN: 0030-9982
- COULIBALY FASSELI ET AL: "The birnavirus crystal structure reveals structural relationships among icosahedral viruses", CELL, vol. 120, no. 6, March 2005 (2005-03), pages 761-772, ISSN: 0092-8674

## Description

The invention relates to therapeutic vaccines for the treatment of neoplasias caused by the human papillomavirus (HPV). In particular, the vaccines of the present disclosure are formed by chimeric virus-like capsids of birnavirus containing papillomavirus antigens.

### PRIOR ART

Human papillomaviruses (HPV) are the main causes of development of intrauterine neoplasms and are frequently associated to other neoplasias such as anal cancer, vulvar cancer, cancer of the penis or oropharyngeal squamous cell carcinoma. HPV infections can go unnoticed for years until they may appear in the form of local neoplasias in varying degrees of progression. This is why regular smear tests are performed on women with a view to identify pre-malignant cells and where applicable the type of causing virus. The most common strains of HPV associated to local intrauterine neoplasias are HPV-16 and HPV-18, involved in 70% of all cases of cervical cancer. The oncogenic effect of these HPV is mediated by the integration of part of its genome, in particular of the early expression genes E6 and E7, in the genome of the infected basal epithelial cells.

There are currently prophylactic vaccines on the market to prevent infection by the papillomavirus such as Gardasil® and Cervarix® for example. These vaccines are effective in preventing infection by human papillomavirus strains HPV-16, HPV-18, HPV-11 and HPV-6, but their efficiency in a therapeutic context has not been proven once the HPV has transformed the infected cell into a pre-cancerous cell.

It is estimated that in just the US, 10 million women are in contact with HPV every year and it is predicted that 23 % of these women will develop local intrauterine neoplasias (or CIN, Cervical Intrauterine Neoplasias) to some degree, at some point in their life [Hoory T el al. (2008) J. Formos Med. Assoc 107(3):198-217]. Furthermore, every year 275,000 women die in the world due to cervical cancer as a result of the unfavourable development of a CIN. Although currently cervical cancer prevention and monitoring programmes by means of regular gynaecological check-ups and surgical removal of CIN are effective, it would be desirable to count on a non-invasive therapeutic approach that would prevent surgery-related costs and potential complications.

There are precedents in the development of therapeutic vaccines for the treatment of CIN that incorporate E6 and/or E7 antigens of the papillomavirus [Lin Y. et al. (2007) Frontier in Bioscience, 12: 246-264; Leggatt GR et al. (2007) Current Opin. Immunology, 19: 232-238; Hung CF et al. (2007) Exp. Mol. Med, 39(6):679-689; Hoory T et al. (2008) J. Formos Med. Assoc, 107(3):198-217]. These include DNA vaccines [Yan Q. et al (2007) Gynecologic Onco., 104:199-206; Yan J. et al. (2009) Vaccine, 27:431-440], vaccines based on naked peptides [Daffarin PM. et al (2007) J. Translational Med., 5:26; Toubaji A. et al. (2007) Vaccine, 25: 5882-5891; Smith KA. et al. (2009) Clin. Cancer Res. 15(19):6167-6176], and vaccines wherein the HPV antigens are linked or fused to other biological entities, such as for example the adenylate cyclase of *Bordetella pertussis* [Preville X. et al. (2005) Cancer Res., 65(2):641-649], antigen 4 of cytotoxic T lymphocytes (CTLA4) [Zheng Y.et al. (2009) J. Microbiol., 46(6):728-736] or 1,3-1,4 beta-glucanase of *Clostridium thermocellum* [Venuti A. et al. (2009) Vaccine, 27 (25-26):3395-3397]. In the case of vaccines using the presentation of antigens on biological macrostructures, one example consists of chimeric virus-like particles (VLP) formed on the basis of structural viral proteins that incorporate antigens relevant to a disease. In this sense there are precedents that incorporate E7 antigens in VLP of Hepatitis B [Tindle RW. et al. Virology (1994) 1;200(2):547-57]. Among the VLP proposed for antigen presentation are those deriving from the Infectious Bursal Disease Virus (IBDV). IBDV belongs to the *Birnaviridae* family and is the agent responsible for causing Gumboro disease in birds. The viral particles of IBDV are icosahedral with a symmetry of T=13 and are formed by 260 trimers of the protein VP2 (37 kDa) reinforced on its internal side by the structural protein VP3 (29 kDa). In the normal assembly of the virion, the protein components of the viral capsid result from proteolysis of the precursor polypeptide pVP2-VP4-VP3 (109 kDa) to release the VP2 precursor of 512 amino acids (pVP2₅₁₂), VP4 and VP3. The subsequent removal of amino acids in the carboxyl terminal of pVP2₅₁₂ gives rise to the mature form of VP2 of 441 amino acids (VP2₄₄₁) present in the virion [Da Costa B. et al. J. Virology (2002) 76(5):2393-2402]. The VP2 found in different strains of IBDV presents a protein sequence homology of more than 80%. The VP2 of other *Birnaviridae* share with IBDV protein sequence homologies of about 40% in the case of aquatic Birnavirus and 30% in Drosophila Birnavirus [Coulibaly F. et al. (2005) Cell 25,120(6):761-772].

The expression of the precursor polypeptide pVP2-VP4-VP3 of IBDV in eukaryotic cells results in the formation of icosahedral VLP with symmetry T=13, identical to the native capsids of IBDV [Martinez-Torrecuadrada JL. et al. (2001) J. Virology 75(22):10815-10828]. At the same time, the expression of VP2 in eukaryotic cells in the absence of other proteins of IBDV results in the formation of icosahedral VLP with symmetry T=1 smaller than those comprising the IBDV [Martinez-Torrecuadrada JL. et al. (2003) Vaccine 21(17-18):1952-1960]. This has been used in the design and expression of chimeric VLP that incorporate the BT antigen of foot and mouth disease in VLP T=1 based on fusions in the carboxyl terminus of VP2 [WO2007009673].

The capacity of fusions or insertions of VP2 to form VLP T=1 efficiently in a eukaryotic expression system depends to a great extent on the length of the VP2 [WO2005105834; Saugar I. et al. (2005) Structure 13(7):1007-1117], on the place of insertion of these inserts in the VP2 sequence, and on the sequence of the inserts introduced in VP2 [Rémond M. et al (2009) Vaccine. 27(1):93-8]. However, the capacity of the obtained chimeric VLP to induce a suitable immune response in an animal model is unpredictable.

### DESCRIPTION OF THE INVENTION

In the design of therapeutic vaccines against HPV it would be desirable to have a HPV antigen presentation system that generated a specific and effective cellular immune response. The present invention combines sequences of VP2 of IBDV, particularly the fragment VP2₄₅₂, and of E7 of HPV, particularly the fragment of SEQ ID NO: 6, with a view to obtaining an effective therapeutic vaccine for the treatment of tumours induced by HPV. It is not obvious which sequences of E7 or VP2 may be ideal in the generation of the vaccine, nor the ideal place of fusion or insertion of E7 with VP2. For this reason, in the present invention a search process is carried out of chimeric VLP T=1 based on fusions and insertions of E7 in VP2 of IBDV resulting in the selection of the most effective chimeric VLP in the treatment of lesions caused by HPV.

The object of the invention is to provide a vaccine that is effective in the treatment of neoplasias caused by the human papillomavirus. The therapeutic vaccines of the present invention consist of chimeric virus-like particles formed on the basis of fusions and insertions of the SEQ ID NO: 6 of the E7 protein of human HPV and the SEQ ID NO: 4 of VP2 of the infectious bursal disease virus. The search and selection process carried out results in viral-like particles based on fusions and insertions of E7_{Δ1-44} of HPV with VP2₄₅₂ of IBDV with greater antitumoral efficacy and object of the present invention.

The present disclosure relates to chimeric virus-like particles (VLP) based on birnavirus VP2 that incorporate sequences of HPV oncogenes. The fusion or insertion of sequences not related to birnavirus in VP2 often results in modifications of the three-dimensional structure of the protein which negatively affect its capacity to self-assemble and form virus-like capsids in an efficient manner. This negative effect not only depends on the insertion point, but also on the amino acid sequence and length of the insert. Therefore it is not obvious *a priori* which insertion points and inserted amino acid sequences result in an efficient formation of chimeric VLP. At the same time, the formation of VLP that incorporate oncogenic sequences of HPV is not a sufficient condition to generate an efficient therapeutic vaccine against tumours, this depends on the final arrangement of the E7 antigens on the formed chimeric VLP.

The chimeric VLP of the present invention are obtained from a selection process wherein the optimum sequence of E7 for the formation of VLP is identified and the places of fusion or insertion in VP2 that give rise to chimeric VLP of greater efficacy in the treatment of tumours expressing oncogenic proteins E6 and E7 of HPV-16.

In the first place, as described without limitation in Example 1, fusions are evaluated of truncated sequences of E7 to the carboxyl terminal end of sequences of VP2 truncated at the carboxyl terminus. Among others, fusions of sequences of E7 are evaluated wherein amino acids 1 to 35 have been eliminated (E7_{Δ1-35}) [SEQ ID NO: 3] to the carboxyl end of the VP2 truncated at the carboxyl end starting from amino acid 452 (VP2₄₅₂) [SEQ ID NO: 4]. In order to improve the expression, various fusions are also evaluated of E7_{Δ1-35} to the carboxyl terminus of VP2₄₄₁ , VP2₄₄₃ , VP2₄₄₆ VP2₄₄₉ and VP2₄₅₀ resulting from the elimination of amino acids from the carboxyl terminal end of VP2₄₅₂ . No case exceeds the efficiency in the production of chimeric VLP of the fusions VP2₄₅₂-E7_{Δ1-35} [SEQ ID NO: 5]. In order to select the sequences of E7 that fused to the carboxyl terminal end of VP2 give rise to a greater expression, fusions are evaluated to the carboxyl terminal end of VP2₄₅₂ of the following truncated E7 sequences: E7_{Δ1-40}, E7_{Δ1-41}, E7_{Δ1-44}. The fusion of truncated E7 E7_{Δ1-44} [SEQ ID NO: 6] with the Arginine (R) of the carboxyl terminal end of VP2₄₅₂ [SEQ ID NO: 4] results in a construction VP2₄₅₂-E7_{Δ1-44} [SEQ ID NO: 7] with an efficiency in the production of chimeric VLP superior to others evaluated. At the same time, VLP with the amino acid sequence VP2₄₅₂-E7_{A1-44} prove to be effective in the elimination of tumours that express proteins E6 and E7 of HPV-16 in animal models and represent a first aspect of the present invention. Therefore, the present invention incorporates chimeric virus-like particles based on the sequence of E7 of HPV-16 from which amino acids 1 to 44 have been eliminated (E7_{Δ1-44}), fused to the carboxyl terminal end of VP2 truncated in the carboxyl end from amino acid 452 (VP2₄₅₂). It is contemplated that these fusions may contain variations or insertions in their amino acid sequence of 7, and up to 10 amino acids, in particular in the points of fusion between VP2₄₅₂ and E7_{Δ1-44} and as a result of the use of cloning sequences.

In second place, and as described without limitation in Example 2, random insertions are evaluated of sequences of E7 in HPV-16 wherein amino acids 1 to 44 have been eliminated (E7_{Δ1-44}) [SEQ ID NO: 6] in different points in the sequence of VP2 truncated in the carboxyl end from amino acid 452 (VP2₄₅₂) [SEQ ID NO: 4]. The process of identification and selection of the candidates with a greater potential for efficacy in the treatment of subcutaneous tumours expressing oncogenic proteins E6 and E7 of HPV-16 is carried out according to the following steps. In a first step, random insertions are made of E7_{Δ1-44} in VP2₄₅₂ and all those constructions that do not result in the efficient expression of VLP are eliminated. In a second step, a selection process is carried out of the candidates that produce VLP efficiently and that prove to contain the sequence of E7_{Δ1-44}. In a third step, those chimeric VLP that generate a significant cellular immune response against protein E7 are selected. In a fourth step, the capacity of the selected chimeric VLP to provide an antitumoral effect in an animal model of neoplasia associated to the expression of E7 is evaluated. As a final result of the process, three chimeric VLP are selected which represent preferred embodiments of the present invention, which are VP2₄₅₂(L₄₃₆↑E7_{Δ1-44}↑K₄₃₇) wherein the sequence of E7_{Δ1-44} is inserted between Leucine (L) in position 436 and Lysine (K) in position 437 of VP2₄₅₂ [SEQ ID NO: 8]; VP2₄₅₂(A₄₄₁↑E7_{Δ1-44}↑F₄₄₂) wherein the sequence of E7_{Δ1-44} is inserted between Alanine (A) in position 441 and Phenylalanine (F) in position 442 of VP2₄₅₂ [SEQ ID NO: 9]; and VP2₄₅₂(A₄₅₀↑E7_{Δ1-44}↑I₄₅₁) wherein the sequence of E7_{Δ1-44} is inserted between Alanine (A) in position 450 and Isoleucine (I) in position 451 of VP2₄₅₂ [SEQ ID NO: 10].

Therefore, the present invention describes chimeric virus-like particles based on a sequence of E7 of HPV-16 wherein amino acids 1 to 44 have been eliminated (E7_{Δ1-44}), inserted in the VP2 truncated in the carboxyl end from amino acid 452 (VP2₄₅₂) in positions L₄₃₆↑K₄₃₇ , A₄₄₁↑F₄₄₂ and A₄₅₀↑I₄₅₁. It is contemplated that these fusions contain variations or insertions in their sequence of up to 15 amino acids at each end of the insert, in particular in the fusion points between VP2₄₅₂ and E7_{Δ1-44} , as a result of the use of cloning sequences and as a result of the addition of linkers that increase the flexibility of the insertion.

A first aspect of the invention relates to a chimeric virus-like particle (VLP) (hereinafter, chimeric VLP of the invention) formed by a fusion protein (hereinafter, fusion protein of the invention) which comprises:
- a subunit (a) consisting of the pVP2 protein fragment of IBDV VP2₄₅₂ of SEQ ID NO: 4, and
- a subunit (b) consisting of SEQ ID NO: 6 of the human papillomavirus (HPV),
wherein subunit (b) is linked to the carboxyl-terminal end of subunit (a) or subunit (b) is inserted in subunit (a).

The term "virus-like capsid", "virus-like particle" or "VLP" refers to a three-dimensional nanometric structure formed by the assembly of structural viral proteins. In the present invention, the structural viral proteins forming the virus-like particle of the invention are fusion proteins that comprise the pVP2 protein fragment of IBDV VP2₄₅₂ of SEQ ID NO: 4 and the SEQ ID NO: 6 of HPV.

The term Birnavirus refers to any virus of the *Birnaviridae* family, belonging to Group III according to the Baltimore Classification. The *Birnaviridae* family consists of the *Avibirnavirus, Aquabirnavirus, Blosnavirus* and *Entomobirnavirus* genuses. In the present invention, the Birnavirus is of the *Avibirnavirus* family, and is the Infectious Bursal Disease Virus (IBDV).

The term "infectious bursal disease virus" or "IBDV" refers to viruses of the *Birnaviridae* family and *Avibirnavirus* genus causing Gumboro disease in chicken and belonging to Group III of the Baltimore Classification. Preferably, the IBDV is the Soroa strain IBDV.

The Birnavirus genome is formed by two linear molecules of double-stranded RNA referred to as A and B, encoding 5 proteins. Gene VP2, embedded in segment A, encodes the precursor protein of protein VP2 (pVP2). The elimination of the carboxyl terminal end of pVP2 by proteolysis gives rise to the mature VP2 protein, which is the main protein the viral capsid consists of.

The term "pVP2 protein" or "pVP2", as used in the present description, refers to the precursor protein of VP2 encoded by the gene *VP2* of a Birnavirus. Preferably, this term refers to the precursor protein of VP2 of 512 amino acids (VP2₅₁₂) of IBDV.

The amino acid sequence of protein VP2₅₁₂ of the Soroa strain IBDV (SEQ ID NO: 1) is deposited with access number AAD30136 in the NCBI (*National Center for Biotechnology Information*). Protein VP2₅₁₂ in other strains of IBDV presents at least 80% identity with SEQ ID NO: 1. Therefore, in a preferred embodiment, the term pVP2 refers to a protein having at least 80%, 85%, 90%, 95%, 98% or 99% identity, with SEQ ID NO: 1. In a more preferred embodiment, the term pVP2 refers to SEQ ID NO: 1.

The term "identity" as used in this description, refers to the proportion of identical amino acids between two compared sequences of amino acids. The percentage of identity that exists between two amino acid sequences can be easily identified by a person skilled in the art, for example using a suitable sequence comparison software.

The term "fragment", as used in the present description, refers to a portion of the pVP2 protein, of at least 400 amino acids, capable of forming VLP. This term therefore includes the mature VP2 protein of 441 amino acids of IBDV (VP2₄₄₁).

In a preferred embodiment of this disclosure, subunit (a) of the fusion protein that forms the chimeric VLP of the invention consists of a protein having at least 80% identity with SEQ ID NO: 1 or a fragment thereof. In a more preferred embodiment of this disclosure, subunit (a) of the fusion protein that forms the chimeric VLP of the invention consists of a protein having SEQ ID NO: 1 or a fragment thereof. In the present invention, subunit (a) of the fusion protein that forms the chimeric VLP of the invention consists of SEQ ID NO: 4.

The term "human papillomavirus" or "HPV" refers to a virus of the *Papillomaviridae* family belonging to Group I of the Baltimore Classification, and therefore having a double-stranded DNA genome. More than 100 different types of human papillomavirus are known. The strains of HPV most frequently associated to intrauterine neoplasia are HPV-16 and HPV-18. The oncogenic effect of these HPV is mediated by the integration of part of its genome, in particular early expression genes E6 and E7, in the genome of the infected basal epithelial cells.

The term "early expression protein E6", "early expression oncogene E6" or "E6" refers to the protein encoded by the early expression gene E6 of a HPV, and more preferably, of HPV-16 or HPV-18.

The term "early expression protein E7", "early expression oncogene E7" or "E7" refers to a protein encoded by the early expression gene E7 of a HPV, and more preferably, of HPV-16 or HPV-18.

The prototypical sequence of amino acids of the early expression protein E7 of HPV type 16 [SEQ ID NO: 2] is deposited with access number NP_041326 in the NCBI. Therefore, in a preferred embodiment of this disclosure, the term early expression protein E7 refers to a protein having at least 30%, 40%, 50%, 70%, 90%, 95%, 98%, or 99% identity, with SEQ ID NO: 2. In a more preferred embodiment of this disclosure, the term early expression protein E7 of HPV refers to SEQ ID NO: 2.

In another preferred embodiment of this disclosure, subunit (b) of the fusion protein that forms the chimeric VLP of the invention consists of a protein having at least 30% identity with SEQ ID NO: 2 or a fragment thereof. In a more preferred embodiment of this disclosure, subunit (b) of the fusion protein that forms the chimeric VLP of the invention consists of a protein with SEQ ID NO: 2 or a fragment thereof. In the present invention, subunit (b) of the fusion protein that forms the chimeric VLP of the invention consists of SEQ ID NO: 6.

In this first aspect of the invention, subunit (b) may be joined to the carboxyl-terminal end of the subunit (a) in order to give rise to the fusion protein that forms the chimeric VLP of the invention. This link may be direct or by means of a linker polypeptide.

The term "linker polypeptide" or "linker" as used in the present description, refers to a short amino acid sequence, preferably, of up to 20 amino acids in length, more preferably, of up to 15 amino acids in length, and even more preferably, of up to 10 amino acids in length, situated between the sequence of amino acids of subunit (b) and the sequence of amino acids of subunit (a).

When the link is direct between subunits (a) and (b) of the fusion protein that forms the VLP of the invention, the amino acid of the carboxyl-terminal end of subunit (a) forms a peptide bond with the amino acid of the amino-terminal end of subunit (b), as shown in the following diagram:

**Nt-(a)-Ct ∼ Nt-(b)-Ct**

wherein (a) represents subunit (a), (b) represents subunit (b), Nt represents the amino-terminal end of the corresponding subunit, Ct represents the carboxyl-terminal end of the corresponding subunit, and ∼ represents a peptide bond between the different units of the fusion protein of the invention.

When the bond between subunits (a) and (b) of the fusion protein that forms the VLP of the invention is carried out using a polypeptide linker, the amino acid of the carboxyl-terminal end of subunit (a) forms a peptide bond with the amino acid of the amino-terminal end of the linker peptide (p) and the amino acid of the carboxyl-terminal end of the linker polypeptide forms a bond with the amino acid of the amino-terminal end of subunit (b), as shown in the following diagram:

**Nt-(a)-Ct ∼ Nt-(p)-Ct ∼ Nt-(b)-Ct**

wherein (a) represents subunit (a), (b) represents subunit (b), (p) represents the linker polypeptide, Nt represents the amino-terminal end of the corresponding subunit or linker polypeptide, Ct represents the carboxyl-terminal end of the corresponding subunit or linker polypeptide, and ∼ represents a peptide bond between the different units of the fusion protein of the invention.

In this first aspect of the invention, subunit (b) may be alternatively inserted in subunit (a) to give rise to the fusion protein that forms the chimeric VLP of the invention. The expression "inserted" means that the sequence of amino acids of subunit (a) is divided into two parts (a1) and (a2), between which the sequence of amino acids of subunit (b) is found.

When the bond between subunits (a) and (b) of the fusion protein that forms the VLP of the invention is direct, the amino acid of the carboxyl-terminal end of part (a1) of subunit (a) forms a peptide bond with the amino acid of the amino-terminal end of subunit (b) and the amino acid of the carboxyl-terminal end of subunit (b) forms a peptide bond with the amino acid of the amino-terminal end of part (a2) of subunit (a), as shown in the following diagram:

**Nt-(a1) -Ct** ∼ **Nt-(b)-Ct** ∼ **Nt-(a2) -Ct**

wherein (a1) represents a part of subunit (a), (a2) represents the other part of subunit (a), (b) represents subunit (b), Nt represents the amino-terminal end of the corresponding subunit, Ct represents the carboxyl-terminal end of the corresponding subunit, and ∼ represents a peptide bond between the different units of the fusion protein of the invention.

When the bond between subunits (a) and (b) of the fusion protein that forms the VLP of the invention is achieved by means of two linker polypeptides, which may be identical or different, the amino acid of the carboxyl-terminal end of part (a1) of subunit (a) forms a peptide bond with the amino acid of the amino-terminal end of a first linker polypeptide (p1), the amino acid of the carboxyl-terminal end of this first linker polypeptide (p1) forms a bond with the amino acid of the amino-terminal end of subunit (b), the amino acid of the carboxyl-terminal end of subunit (b) forms a peptide bond with the amino acid of the amino-terminal end of a second linker polypeptide (p2) and the amino acid of the carboxyl-terminal end of this second linker polypeptide (p2) forms a bond with the amino acid of the amino-terminal end of part (a2) of subunit (a), as shown in the following diagram:

**Nt-(a1) -Ct** ∼ **Nt-(p1)-Ct** ∼ **Nt-(b)-Ct** ∼ **Nt-(p2)-Ct** ∼ **Nt-(a2) -Ct**

wherein (a1) represents a part of subunit (a), (a2) represents the other part of subunit (a), (b) represents subunit (b), (p1) represents a first linker polypeptide, (p2) represents a second linker polypeptide, Nt represents the amino-terminal end of the corresponding subunit or linker polypeptide, Ct represents the carboxyl-terminal end of the corresponding subunit or linker polypeptide, and ∼ represents a peptide bond between the different units of the fusion protein of the invention.

When the bond between subunits (a) and (b) of the fusion protein that forms the VLP of the invention is achieved by means of one linker polypeptide only, subunit (b) is linked at one end with one of the parts of subunit (a) directly by means of a peptide bond, and at the other end is linked with the other part of subunit (a) by means of a linker polypeptide, as shown in the following diagrams:

**Nt-(a1) -Ct** ∼ **Nt-(b)-Ct** ∼ **Nt-(p)-Ct** ∼ **Nt-(a2) -Ct**

**Nt-(a1) -Ct** ∼ **Nt-(p)-Ct** ∼ **Nt-(b)-Ct** ∼ **Nt-(a2) -Ct**

wherein (a1) represents one part of subunit (a), (a2) represents the other part of subunit (a), (b) represents subunit (b), (p) represents a linker polypeptide, Nt represents the amino-terminal end of the corresponding subunit or linker polypeptide, Ct represents the carboxyl-terminal end of the corresponding subunit or linker polypeptide, and ∼ represents a peptide bond between the different units of the fusion protein of the invention.

This first aspect of the invention, relates therefore to a chimeric VLP formed by a fusion protein comprising SEQ ID NO: 4 and SEQ ID NO: 6, wherein SEQ ID NO: 6 is linked to the carboxyl-terminal end of SEQ ID NO: 4 or SEQ ID NO: 6 is inserted in SEQ ID NO: 4. A preferred embodiment, relates to a chimeric VLP formed by a fusion protein comprising SEQ ID NO: 4 and SEQ ID NO: 6, wherein SEQ ID NO: 6 is linked to the carboxyl-terminal end of SEQ ID NO: 4 or SEQ ID NO: 6 is inserted in SEQ ID NO: 4 and wherein when SEQ ID NO: 6 is inserted in SEQ ID NO: 4 the fusion protein additionally comprises one or two linker polypeptides of up to 15 amino acids situated between the amino acid sequence of SEQ ID NO: 6 and the amino acid sequence of SEQ ID NO: 4.

A more preferred embodiment of this first aspect of the invention, relates to a chimeric VLP formed by a fusion protein comprising SEQ ID NO: 4 and SEQ ID NO: 6, wherein SEQ ID NO: 6 is linked to the carboxyl-terminal end of SEQ ID NO: 4 by means of a peptide bond between the Arginine of position 452 (R₄₅₂) of SEQ ID NO: 4 and the Alanine of position 1 (A1) of SEQ ID NO: 6.

Another more preferred embodiment of this first aspect of the invention, relates to a chimeric VLP formed by a fusion protein comprising SEQ ID NO: 4 and SEQ ID NO: 6, wherein SEQ ID NO: 6 is linked to the carboxyl end of SEQ ID NO: 4, and additionally comprising a linker polypeptide of up to 10 amino acids between SEQ ID NO: 4 and SEQ ID NO: 6. This linker polypeptide is linked by its amino-terminal end with the Arginine of position 452 (R₄₅₂) of SEQ ID NO: 4 and by its carboxyl-terminal end with the Alanine of position 1 (A1) of SEQ ID NO: 6. An even more preferred embodiment, relates to a chimeric VLP formed by the fusion protein whose amino acid sequence is SEQ ID NO: 7.

Another preferred embodiment of this first aspect of the invention, relates to a chimeric VLP formed by a fusion protein comprising SEQ ID NO: 4 and SEQ ID NO: 6, wherein SEQ ID NO: 6 is inserted in SEQ ID NO: 4. As a result of the insertion of SEQ ID NO: 6, SEQ ID NO: 4 is divided into two parts. A more preferred embodiment, relates to a chimeric VLP formed by a fusion protein comprising SEQ ID NO: 4 and SEQ ID NO: 6, wherein SEQ ID NO: 6 is inserted in SEQ ID NO: 4, and additionally comprising one or two linker polypeptides of up to 15 amino acids each one situated between the amino acid sequence of SEQ ID NO: 6 and the amino acid sequences of the two parts into which SEQ ID NO: 4 is divided as a result of the insertion.

A preferred embodiment of this first aspect of the invention, relates to a chimeric VLP formed by a fusion protein comprising SEQ ID NO: 4 and SEQ ID NO: 6, wherein SEQ ID NO: 6 is inserted between the Leucine of position 436 (L₄₃₆) and the Lysine of position 437 (K₄₃₇) of SEQ ID NO: 4. A more preferred embodiment, relates to a chimeric VLP formed by a fusion protein comprising SEQ ID NO: 4 and SEQ ID NO: 6, wherein SEQ ID NO: 6 is inserted between amino acids L₄₃₆ and K₄₃₇ of SEQ ID NO: 4, and additionally comprising one or two linker polypeptides of up to 15 amino acids each one situated between the amino acid sequence of SEQ ID NO: 6 and the amino acid sequences of the two parts into which SEQ ID NO: 4 is divided as a result of the insertion. An even more preferred embodiment of this first aspect of the invention, relates to a chimeric VLP formed by a fusion protein whose amino acid sequence is SEQ ID NO: 8.

A preferred embodiment, relates to a chimeric VLP formed by a fusion protein comprising SEQ ID NO: 4 and SEQ ID NO: 6, wherein SEQ ID NO: 6 is inserted between the Alanine of position 441 (A₄₄₁) and the Phenylalanine of position 442 (F₄₄₂) of SEQ ID NO: 4. A more preferred embodiment, relates to a chimeric VLP formed by a fusion protein comprising SEQ ID NO: 4 and SEQ ID NO: 6, wherein SEQ ID NO: 6 is inserted between amino acids A₄₄₁ and F₄₄₂ of SEQ ID NO: 4, and additionally comprising one or two linker polypeptides of up to 15 amino acids each one situated between the amino acid sequence of SEQ ID NO: 6 and the amino acid sequences of the two parts into which SEQ ID NO: 4 is divided as a result of the insertion. An even more preferred embodiment of this first aspect of the invention, relates to a chimeric VLP formed by a fusion protein whose amino acid sequence is SEQ ID NO: 9.

A preferred embodiment, relates to a chimeric VLP formed by a fusion protein comprising SEQ ID NO: 4 and SEQ ID NO: 6, wherein SEQ ID NO: 6 is inserted between the Alanine of position 450 (A₄₅₀) and the Isoleucine of position 451 (I₄₅₁) of SEQ ID NO: 4. A more preferred embodiment, relates to a chimeric VLP formed by a fusion protein comprising SEQ ID NO: 4 and SEQ ID NO: 6, wherein SEQ ID NO: 6 is inserted between amino acids A₄₅₀ and I₄₅₁ of SEQ ID NO: 4, and additionally comprising one or two linker polypeptides of up to 15 amino acids each one situated between the amino acid sequence of SEQ ID NO: 6 and the amino acid sequences of the two parts into which SEQ ID NO: 4 is divided as a result of the insertion. An even more preferred embodiment of this first aspect of the invention, relates to a chimeric VLP formed by a fusion protein whose amino acid sequence is SEQ ID NO: 10.

A second aspect of the invention relates to a process for obtaining the chimeric VLP of the invention, which comprises cultivating a host cell that comprises a nucleic acid encoding the fusion protein of the invention, under conditions that allow the expression of said fusion protein, and the assembly of said fusion protein to form chimeric VLP.

A preferred embodiment of this second aspect of the invention, relates to a process for obtaining the chimeric VLP particles of the invention, which comprises cultivating a host cell that comprises a nucleic acid encoding the fusion protein of the invention, under conditions that allow the expression of said fusion protein, and the assembly of said fusion protein to form chimeric VLP, and which additionally comprises isolating or purifying said chimeric VLP.

The fusion protein of the invention may be obtained by means of recombinant or genetic engineering techniques well known in the state of the art. The sequence of a nucleic acid that encodes the fusion protein of the invention (hereinafter, nucleic acid of the disclosure) may be obtained by means of any synthetic or biological method, for example, but without limitation, the restriction of suitable sequences or the amplification of the DNA sequence of the protein of interest through polymerase chain reaction (PCR).

The nucleic acid may be comprised in a gene construct (hereinafter gene construct of the disclosure). This gene construct of the disclosure may comprise the nucleic acid of the disclosure, operatively linked to a sequence regulating the expression of the nucleic acid of the disclosure, thereby constituting an expression *cassette.*

"Operatively linked" refers to a juxtaposition wherein the components thus described have a relationship that allows them to function in the intended manner. A control sequence "operatively linked" to the nucleic acid, is linked thereto in such a way that the expression of the sequence encoding the nucleic acid is achieved.

"Control sequence" refers to sequences of nucleic acids that affect the expression of the sequences whereto they are linked. Said control sequences include, for example, but without limitation, promoters, initiation signals, termination signals, intensifiers or silencers. The term "control sequences" is intended to include those components whose presence is necessary for the expression, and may also include additional components whose presence is advantageous.

In a preferred embodiment, the gene construct of the disclosure comprises the nucleic acid of the disclosure operatively linked to, at least, one control sequence of the list that comprises:
a. a promoter,
b. a transcription initiation signal,
c. a transcription termination signal,
d. a polyadenylation signal, or
e. a transcriptional activator.

As used herein, the term "promoter" refers to a region of DNA situated in position 5' in respect of the transcription initiation point and that is necessary or facilitates said transcription in an animal cell. This term includes, for example, but without limitation, constituent promoters, specific promoters of the cell type or of tissue or inducible or repressible promoters.

The control sequences depend on the origin of the cell wherein the nucleic acid of the disclosure is to be expressed. In a particular embodiment, the expression control sequences linked to the nucleic acid of the disclosure are functional in prokaryote organisms and cells, for example, but without limitation, bacteria; whereas in another particular embodiment, said expression control sequences are functional in eukaryote organisms and cells, for example, yeast cells or animal cells.

The nucleic acid of the disclosure or gene construct of the disclosure may be introduced inside a cell, referred to as a host cell, for example, but without limitation, in the form of a naked nucleic acid or by means of a vector.

The term "cloning vector", as used in the present description, refers to a molecule of DNA wherein another fragment of DNA may be integrated, without it losing the capacity to self-replicate. Examples of expression vectors are, but without limitation, plasmids, cosmids, DNA phages or artificial yeast chromosomes.

The term "expression vector", as used in the present description, refers to a cloning vector suitable for expressing a nucleic acid that has been cloned therein after being introduced in a cell, referred to as the host cell. Said nucleic acid is, in general, operatively linked to control sequences.

The term "host cell", as used in the present description, refers to any prokaryote or eukaryote organism that is the receiver of an expression vector, cloning vector or any other DNA molecule.

A third aspect of the invention relates to the use of the chimeric VLP of the invention in the production of a drug, preferably, a vaccine.

Another aspect of the disclosure relates to the use of the chimeric VLP of the invention in the production of a drug for the prevention and/or treatment of an infection caused by HPV, preferably, HPV-16.

A fourth aspect of the invention relates to the use of the chimeric VLP of the invention in the production of a drug for the prevention and/or treatment of a neoplasia caused by the human papillomavirus.

A fifth aspect of the invention relates to the use of the chimeric VLP of the invention in the production of a drug for the prevention and/or treatment of cervical cancer.

A sixth aspect of the invention relates to a pharmaceutical composition (hereinafter, pharmaceutical composition of the invention) comprising the chimeric VLP of the invention.

A preferred embodiment of this sixth aspect of the invention relates to a pharmaceutical composition comprising the chimeric VLP of the invention and additionally comprising a pharmaceutically acceptable vehicle. Another preferred embodiment of this disclosure relates to a pharmaceutical composition comprising the chimeric VLP of the invention and additionally comprising another active principle. A more preferred embodiment of this disclosure relates to a pharmaceutical composition comprising the chimeric VLP of the invention, a pharmaceutically acceptable vehicle and additionally another active principle.

As used herein, the terms "active principle", "active substance", "pharmaceutically active substance", "active ingredient", or "pharmaceutically active ingredient" refer to any component which potentially provides pharmacological activity or other different effect on the diagnosis, cure, mitigation, treatment or prevention of a disease, or which affects the structure or function of the body of humans or other animals.

The pharmaceutical composition of the invention may be formulated for administration through a variety of forms known in the state of the art. Such formulations may be administered to an animal, and more preferably, to a mammal, including a human being, through a variety of routes, including, but without limitation, parenteral, intraperitoneal, intravenous, intradermal, epidural, intraspinal, intrastromal, intra-articular, intrasynovial, intrathecal, intralesional, intra-arterial, intracapsular, intracardiac, intramuscular, intranasal, intracraneal, subcutaneous, intraorbital, intracapsular or topical.

The dose for obtaining a therapeutically effective amount will depend on a variety of factors, such as, for example, age, weight, sex or tolerance of the animal. In the sense used in this description, the expression "therapeutically effective amount" refers to the amount of the pharmaceutically effective composition that produces the required effect and, in general, will be determined, among other causes, by the characteristics inherent to the pharmaceutical composition in question and the therapeutic effect to be achieved. The pharmaceutically acceptable "adjuvants" or "vehicles" that may be used in such composition are the vehicles known in the state of the art.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### DESCRIPTION OF THE DRAWINGS

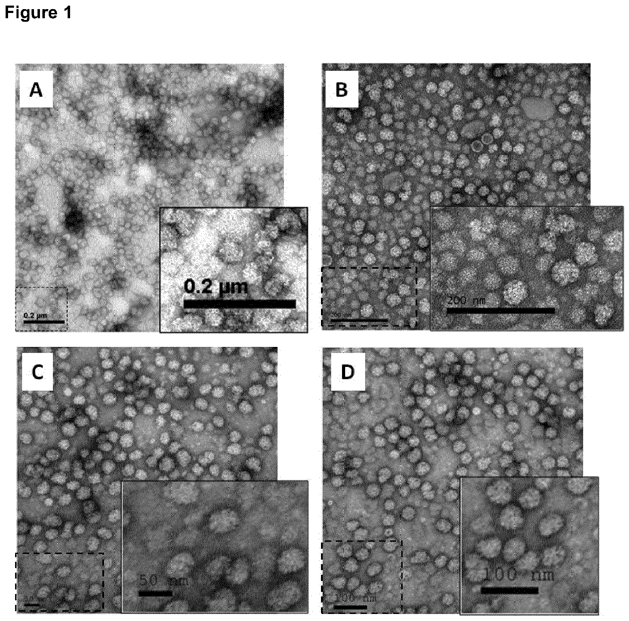

**Figure 1****.** Shows the microphotographs by scanning electron microscope of the virus-like particles resulting from the expression fusion proteins a) VP2₄₅₂-E7_{Δ1-44}; *b)* VP2₄₅₂(L₄₃₆↑E7_{Δ1-44}↑K₄₃₇); *c)* VP2₄₅₂(A₄₄₁↑E7_{Δ1-44}↑F₄₄₂); *d)* VP2₄₅₂(A₄₅₀↑E7 _{Δ1-44↑}I₄₅₁). The dotted line in each photograph (left, bottom) corresponds to the amplified part. The scale bars appear on each photograph.

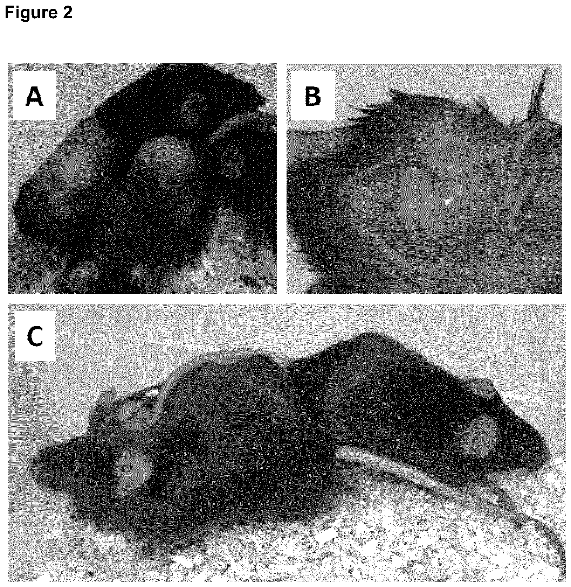

**Figure 2****.** C57BL/6-TgN(HLA-A2.1) mice after 25 days of being xenotransplanted subcutaneously with TC1/A2 cells: (A) mice vaccinated with control VLP (VP2₄₅₂) showing tumours of approximately 1 cm³; (B) necropsy of mouse vaccinated with control VLP (VP2₄₅₂); (C) representative photo of mice vaccinated with VLP containing sequences of E7 and that are tumour-free.

### EXAMPLES

The following specific examples provided in this patent document serve to illustrate the nature of the present invention. These examples are included for solely illustrative purposes and are not to be interpreted as limitations of the invention claimed herein. Therefore, the examples described below illustrate the invention without limiting the field of application thereof.

### EXAMPLE 1: SEARCH AND SELECTION, BY MEANS OF A SUCCESSIVE SCREENING PROCESS, OF CHIMERIC VLP BASED ON CARBOXYL-TERMINALS FUSIONS OF VP2 WITH E7 SEQUENCES OF HPV.

With a view to identifying chimeric virus-like particles effective in the treatment of neoplasias caused by HPV different constructs are generated incorporating the VP2 protein truncated in different points of its carboxyl terminal and different E7 sequences of HPV. To this effect, DNA constructs are produced in the expression plasmid pESC-URA (Stratagene™) which express VP2 truncated in their carboxyl end in the amino acids in position 452, 450, 449, 446, 443, 441 (VP2₄₅₂ , VP2₄₅₀ , VP2₄₄₉ , VP2₄₄₆ , VP2₄₄₃ , VP2₄₄₁), and incorporating restriction sites *NotI* and *HindIII* in the carboxyl terminal end pESC-URA/ VP2₄₅₂ *NotI-HindIII;* pESC-URA/ VP2₄₅₀ *Not*I*-Hind*III*;* pESC-URA/ VP2₄₄₉ *NotI-HindIII*; pESC-URA/ VP2₄₄₆ *NotI-HindIII*; pESC-URA/ VP2₄₄₃ *NotI-HindIII*; and pESC-URA/ VP2₄₄₁ *NotI-HindIII.* Following the *NotI* - *HindIII* double digestion in the carboxyl terminal of the different VP2 a sequence is cloned expressing the E7 protein of HPV-16 wherein the amino acid sequences 1 to 35 (E7Δ₁₋₃₅) associated to its oncogenicity have been eliminated. As shown in Table 1, the capacity to produce VLP in *Saccharomyces cerevisiae* Y449 transformed with the different resulting expression vectors, pESC-URA/ VP2₄₅₂-E7Δ₁₋₃₅ to express fusion protein VP2₄₅₂-E7Δ₁₋₃₅ [SEQ ID NO: 5], pESC-URA/ VP2₄₅₀-E7Δ₁₋₃₅ to express fusion protein VP2₄₅₀-E7Δ₁₋₃₅ [SEQ ID NO: 11], pESC-URA/ VP2₄₄₉-E7Δ₁₋₃₅ to express fusion protein VP2₄₄₉-E7Δ₁₋₃₅ [SEQ ID NO: 12], pESC-URA/ VP2₄₄₆-E7Δ₁₋₃₅ to express fusion protein VP2₄₄₆-E7Δ₁₋₃₅ [SEQ ID NO: 13], pESC-URA/ VP2₄₄₃-E7Δ₁₋₃₅ to express fusion protein VP2₄₄₃-E7Δ₁₋₃₅ [SEQ ID NO: 14], pESC-URA/ VP2₄₄₁-E7Δ₁₋₃₅ to express fusion protein VP2₄₄₁-E7Δ₁₋₃₅ [SEQ ID NO: 15], is compared to that of pESC-URA/ VP2₄₅₂ to express protein VP2₄₅₂ [SEQ ID NO: 4]. As part of the same experiment fusions are also generated to the carboxyl terminal of VP2₄₅₂ based on the double digestion *Not*I - *Hind*III of pESC-URA/ VP2₄₅₂ *NotI-HindIII* and cloning of different sequences expressing the E7 protein of HPV-16 wherein amino acids 1-40 (E7_{Δ1-40}) have been eliminated to express the fusion protein VP2₄₅₂-E7Δ₁₋₄₀ [SEQ ID NO: 16], amino acids 1-41 (HPV16 E7_{Δ1-41}) to express the fusion protein VP2₄₅₂-E7Δ₁₋₄₁ [SEQ ID NO: 17], and amino acids 1-44 (HPV16 E7_{Δ1}-₄₄) to express the fusion protein VP2₄₅₂-E7Δ₁₋₄₄ [SEQ ID NO: 7]. As shown in Table 1, the capacity to produce VLP in *S. cerevisiae* Y449 transformed with the different resulting expression vectors, pESC-URA/ VP2₄₅₂-E7_{Δ1-40}, pESC-URA/ VP2₄₅₂-E7_{Δ1-41} or pESC-URA/ VP2₄₅₂-E7_{Δ1-44} is compared to that of pESC-URA/ VP2₄₅₂ *NotI-HindIII.* The capacity of the different constructs to produce VLP is determined by means of a conformational antibody that recognises the three-dimensional structure of the VLP and subsequently analysed by means of an ELISA-type immunoassay. At the same time, the production and purification of the chimeric VLP and control VLP for morphological studies and *in vivo* tests is carried out following a standard process for yeast culture during 48 hours, concentration through centrifugation, mechanical lysing, precipitation in ammonium sulphate, purification by means of gel filtration chromatography. In all cases, the presence of VLP is confirmed by electron microscope and the chimeric VLP produced are quantified through protein electrophoresis in polyacrylamide gel under denaturing conditions.

The performance in the production of the chimeric VLP evaluated is set out in Table 1. As the next step in the selection process, the capacity to induce an immune response is evaluated of the VLP resulting from the expression of the constructs with VLP production efficiency comparable or superior to the control VP2₄₅₂-E7_{Δ1-35}. In summary, the ELISPOT assay is aimed at measuring the induction of response in cytotoxic T lymphocytes (CTL) in a mouse model. To this effect transgenic C57BL/6-TgN(HLA-A2.1)1Enge/J mice are used, humanized with the histocompatibility complex HLA-A2. The mice are inoculated in groups of eight by means of subcutaneous administration of the chimeric VLP on days 0 and 14. On day 20 the animals are sacrificed, spleens are removed and splenocytes are isolated. Once isolated, the splenocytes are cultivated in the presence of IL-2 and subsequently stimulated with specific peptides (T epitopes) of protein E7 of HPV-16 during 24 hours. Following incubation, the expression of IFN-γ of the clones of CTLs is evaluated as a measurement of their activation. The capacity to induce a specific CTL response against T epitopes of protein E7 of HPV-16 of each one of the evaluated chimeric VLP is set out in Table 1 wherein the number of "+" represents the intensity of the induced response.

As a final step in the selection process, the antitumoral activity of the chimeric VLP with the highest values of performance in VLP production and the best results in the ELISPOT assay is evaluated. To evaluate the antitumoral activity a cell and animal model of tumour induction is used wherein 5x10⁵ cells TC1/A2 [according to Peng S. et al. Gene Therapy, 13:257-265 (2006)] over-expressing tumoral antigens E6 and E7 of HPV-16 are implanted subcutaneously in C57BL/6-TgN(HLA-A2.1)1Enge/J mice humanized with the histocompatibility complex HLA-A2. The animals are divided into groups of ten and each animal is administered 50 ug of the chimeric or control VLP through subcutaneous administration on days 5 and 12 after induction of the tumour. Periodically, tumour development is determined and the weight of the animals is monitored. The animals with tumours having a volume in excess of 1cm³ are sacrificed. The therapeutic effect of the chimeric VLP on tumour model TC1/A2 is set out in Table 1 which shows the percentage of animals that survived 60 days after induction of the tumour.

As a final result of this process, the chimeric VLP is selected containing construct VP2₄₅₂-E7_{Δ1-44}, wherein the sequence of truncated E7 E7_{Δ1-44} is fused to the carboxyl terminal end of VP2 in the Arginine (R) of position 452.

**Table 1**

| **Clone** | **Identification** | **% VLP** | **ELISPOT** | **Antitumoral effect Survival %** |
|---|---|---|---|---|
| 1 | VP2₄₅₂-E7_{Δ1-35} | 20 % | +++ | 80 % |
| 2 | VP2₄₅₀-E7_{A1-35} | 16 % | ND | ND |
| 3 | VP2₄₄₉-E7_{Δ1-35} | 20 % | +++ | ND |
| 4 | VP2₄₄₆-E7_{Δ1-35} | 14 % | ND | ND |
| 5 | VP2₄₄₃-E7_{Δ1-35} | 8 % | ND | ND |
| 6 | VP2₄₄₁-E7_{Δ1-35} | 16 % | ++ | ND |
| 7 | VP2₄₅₂-E7_{Δ1-40} | 25 % | +++ | ND |
| 8 | VP2₄₅₂-E7_{Δ1-41} | 25 % | +++ | ND |
| 9 | VP2₄₅₂-E7_{Δ1-44} | 40 % | ++++ | 100 % |
| 10 | VP2₄₅₂ | 100 % | - | 0 % |

| | | | | |
|---|---|---|---|---|
| %VLP: Performance in the production of VLP compared to VP2₄₅₂; ND: Not determined. | | | | |

### EXAMPLE 2: CLONING, SEARCH AND SELECTION, BY MEANS OF A SUCCESSIVE SCREENING PROCESS, OF CHIMERIC VLP WITH THERAPEUTIC EFFICACY AGAINST NEOPLASIAS CAUSED BY THE HUMAN PAPILLOMAVIRUS.

In a first step, the process is carried out of random insertion in the VP2 gene of the sequences encoding non-transforming regions of E7 wherein amino acids 1 to 44 (E7_{Δ1-44}) have been deleted. For this a library is prepared of VP2 truncated in the carboxyl end as of amino acid 452 (VP2₄₅₂), which contains random insertions of transposon Mu throughout its sequence. In a next step, the insert of transposon Mu is replaced by an insert [SEQ ID NO: 18] containing the kanamycin resistance gene and unique restriction sites *Not*I and *SpeI* at its ends. The cloned insert facilitates the incorporation of inserts E7_{Δ1-44} [SEQ ID NO: 19] or E7_{Δ1-44}(linker) [SEQ ID NO: 20] of HPV-16 synthesized to contain ends *Bsp120*I (compatible with *Not*I) and *SpeI* for cloning in each one of the three possible reading frames. The fusion of inserts E7_{Δ1-44} or E7_{Δ1-44}(linker) generates additional amino acids at the point of fusion between VP2₄₅₂ and E7_{Δ1-44}. The insert E7_{Δ1-44}(linker) additionally contains sequences GGGGS [SEQ ID NO: 21] at the two ends of E7_{Δ1-44} that have been introduced in order to increase the flexibility of insertion (*"linkers"*)*.*

Following the double digestion of the library with *Not*I and *SpeI* and the link in the presence of the fragments with ends *Bsp 120*I and *SpeI* of E7_{Δ1-44} or E7_{Δ1-44}(linker), electro-competent cells of *E.coli* are transformed in order to obtain a library of VP2₄₅₂ containing random insertions of E7_{Δ1-44}. The library is expanded and 10 ug of the DNA obtained from it is used to transform *S.cerevisiae* Y449 which is then seeded on Petri dishes containing the medium YNB/CSM-URA with 2% glucose. The approximately 10,000 clones obtained are transferred to selection Petri dishes containing galactose. The colonies obtained from the selection are transferred to PVDF membranes for colony immunoblot identification of those that express VP2 and E7.

In a second step, a selection process is carried out of the recombinant yeast clones whose performance in VLP production is effective and which contain E7_{Δ1-44}. To this effect, cultures are prepared in conditions of expression of fifty clones with the highest signals in the colony immunoblot against VP2 and E7 and after 48 hours of incubation protein extracts are prepared wherein the amount of VLP is quantified by means of an ELISA-type assay that incorporates an antibody which specifically recognises the three-dimensional structure of the VLP formed by VP2. In this first screening, those constructs are selected with the highest performances in VLP production. The presence of the complete sequence of E7_{Δ1-44} and its place of insertion is verified through sequencing of a DNA extract of each one of the selected clones. As set out in Table 2, in this way 12 clones are identified expressing VLP of VP2₄₅₂ containing insert E7_{Δ1-44} and having a production performance compared with the control VLP of (VP2₄₅₂), of between 6 and 20%.

At the same time, the production and purification of the chimeric VLP and control VLP for morphological studies and for *in vivo* tests is carried out following a standard yeast culture process during 48 hours, concentration by means of centrifugation, mechanical lysing, precipitation in ammonium sulphate, purification by means of gel filtration chromatography. In all cases the presence of VLP is confirmed through electron microscopy and the chimeric VLP produced are quantified by means of protein electrophoresis in polyacrylamide gel under denaturing conditions.

In a third step, the chimeric VLP that generate a significant immune response against E7 are selected. For this purpose, the capacity to induce an immune response of the chimeric VLP originated by those constructs having the highest production performance is evaluated. In summary, the ELISPOT assay is aimed at measuring the induction of a cytotoxic T lymphocyte (CTL) response in a mouse model. To this effect, transgenic C57BL/6-TgN(HLA-A2.1)1Enge/J mice are used "humanized" with histocompatibility complex HLA-A2. The mice are inoculated in groups of eight by subcutaneous administration of the chimeric VLP on days 0 and 14. On day 20 the animals are sacrificed, spleens are extracted and splenocytes isolated. Once isolated, the splenocytes are cultivated in the presence of IL-2 and subsequently stimulated with specific peptides (T epitopes) of protein E7 of HPV-16 during 24 hours. Following incubation, the expression of IFN-γ of the CTL clones is evaluated as a measurement of their activation. The capacity to induce a specific CTL response against the epitopes of protein E7 of HPV-16 of each one of the evaluated chimeric VLP is set out in Table 1 wherein the number of "+" represents the intensity of the induced response.

In a fourth step in the selection process, the antitumoral activity of the chimeric VLP having the highest VLP production performances and the best results in the ELISPOT assay is evaluated. For the evaluation of the antitumoral activity a cell and animal model of tumour induction is used wherein 5x10⁵ cells TC1/A2 [according to Peng S. et al. Gene Therapy, 13:257-265 (2006)] over-expressing tumoral antigens E6 and E7 of HPV-16, are implanted subcutaneously in C57BL/6-TgN(HLA-A2.1)1Enge/J mice humanized with histocompatibility complex HLA-A2. The animals are divided into groups of eight and each animal is administered 50 ug of the chimeric or control VLP, via subcutaneous administration on days 5 and 12 after tumour induction. Periodically, tumour development is determined and the weight of the animals is monitored. Animals with tumours of a volume higher than 1cm³ are sacrificed. The therapeutic effect of the chimeric VLP on tumour model TC1/A2 is set out in Table 2 which shows the percentage of animals that survived 60 days after tumour induction.

As the final result of this process, three chimeric VLP object of the present invention are selected, in other words:
a) VP2₄₅₂(L₄₃₆↑E7_{Δ1-44}↑K₄₃₇) [SEQ ID NO: 8]: chimeric VLP wherein the E7_{Δ1-44} sequence is inserted between the Leucine in position 436 and the Lysine in position 437 of the VP2 of 452 amino acids in length;
b) VP2₄₅₂(A₄₄₁↑E7_{Δ1-44}↑F₄₄₂) [SEQ ID NO: 9]: chimeric VLP wherein the E7_{Δ1-44} sequence is inserted between the Alanine in position 441 and the Phenylalanine in position 442 of the VP2 of 452 amino acids in length; and
c) VP2₄₅₂(A₄₅₀↑E7 _{Δ1-44}↑I₄₅₁) [SEQ ID NO: 10]: chimeric VLP wherein the E7_{Δ1-44} sequence is inserted between the Alanine in position 450 and the Isoleucine in position 451 of the VP2 of 452 amino acids in length.

**Table 2**

| **Clone** | **Identification** | **% VLP** | **ELISPOT Result** | **Antitumoral effect Survival %** |
|---|---|---|---|---|
| 1 | VP2₄₅₂(Q₁₀↑E7_{Δ1-44}↑I₁₁) | 10 % | +++ | 60 % |
| 2 | VP2₄₅₂(D₅₁↑E7_{Δ1-44}↑T₅₂) | 6 % | ND | ND |
| 3 | VP2₄₅₂(T₇₃↑E7_{Δ1-44}↑L₇₄) | 6 % | ND | ND |
| 4 | VP2₄₅₂(A₃₈₀↑E7_{Δ1-44}↑K₃₈₀) | 8 % | ND | ND |
| 5 | VP2₄₅₂(L₄₃₆↑E7_{Δ1-44}↑K₄₃₇) | 10 % | +++++ | 100 % |
| 6 | VP2₄₅₂(A₄₄↑tE7_{Δ1-44}↑F₄₄₂) | 20 % | +++++ | 100 % |
| 7 | VP2₄₅₂(A₄₅₀↑E7_{Δ1-44}↑I₄₅₁) | 18 % | +++++ | 100 % |
| *8* | VP2₄₅₂-E7_{Δ1-44} | 40 % | +++++ | 100 % |
| 9 | VP2₄₅₂ | 100 % | - | 0% |

| | | | | |
|---|---|---|---|---|
| %VLP: Performance in VLP production compared with VP2₄₅₂; ND: Not determined. | | | | |

## Claims

1. Chimeric virus-like particle (VLP) formed by a fusion protein comprising:
- a subunit (a) consisting of the pVP2 protein fragment of IBDV VP2₄₅₂ of SEQ ID NO: 4, and
- a subunit (b) consisting of SEQ ID NO: 6,
wherein subunit (b) is linked to the carboxyl-terminal end of subunit (a) or subunit (b) is inserted in subunit (a).

2. Chimeric virus-like particle according to claim 1, wherein subunit (b) is linked to the carboxyl-terminal end of subunit (a) by means of a linker polypeptide of up to 10 amino acids situated between the amino acid sequences of subunits (a) and (b).

3. Chimeric virus-like particle according to any of claims 1 or 2, wherein SEQ ID NO: 6 is linked to R₄₅₂ of SEQ ID NO: 4.

4. Chimeric virus-like particle according to claim 3, wherein the fusion protein has an amino acid sequence as set forth in SEQ ID NO: 7.

5. Chimeric virus-like particle according to claim 1, **characterized in that** wherein subunit (b) is inserted in subunit (a) the fusion protein additionally comprises one or two linker polypeptides of up to 15 amino acids situated between the amino acid sequence of subunit (b) and the amino acid sequence of subunit (a).

6. Chimeric virus-like particle according to any of claims 1 or 5, wherein SEQ ID NO: 6 is inserted between amino acids L₄₃₆ and K₄₃₇ of SEQ ID NO: 4.

7. Chimeric virus-like particle according to claim 6, wherein the fusion protein has an amino acid sequence as set forth in SEQ ID NO: 8.

8. Chimeric virus-like particle according to any of claims 1 or 5, wherein SEQ ID NO: 6 is inserted between amino acids A₄₄₁ and F₄₄₂ of SEQ ID NO: 4.

9. Chimeric virus-like particle according to claim 8, wherein the fusion protein has an amino acid sequence as set forth in SEQ ID NO: 9.

10. Chimeric virus-like particle according to any of claims 1 or 5, wherein SEQ ID NO: 6 is inserted between amino acids A₄₅₀ and I₄₅₁ of SEQ ID NO: 4.

11. Chimeric virus-like particle according to claim 10, wherein the fusion protein has an amino acid sequence as set forth in SEQ ID NO: 10.

12. Process for obtaining chimeric virus-like particles according to any of claims 1 to 11, which comprises cultivating a host cell that comprises a nucleic acid encoding a fusion protein according to any of claims 1 to 11 under conditions that allow the expression of said fusion protein, and the assembly of said fusion protein in order to form chimeric virus-like particles.

13. Process for obtaining chimeric virus-like particles according to the preceding claim, and which additionally comprises isolating or purifying said chimeric virus-like particles.

14. Use of the chimeric virus-like particle according to any of claims 1 to 11 for the preparation of a drug.

15. Use of the virus-like particle according to any of claims 1 to 11 for the preparation of a drug for the prevention and/or treatment of a neoplasia caused by the human papillomavirus.

16. Use of the virus-like particle according to any of claims 1 to 11 for the preparation of a drug for the prevention and/or treatment of cervical cancer.

17. Pharmaceutical composition comprising the chimeric virus-like particle according to any of claims 1 to 11.

18. Pharmaceutical composition according to claim 17 additionally comprising a pharmaceutically acceptable vehicle.

## Patentansprüche

1. Chimärer virusähnlicher Partikel (VLP), gebildet durch ein Fusionsprotein, umfassend:
- eine Untereinheit (a), bestehend aus dem pVP2-Proteinfragment von IBDV VP2₄₅₂ der SEQ ID NO: 4 und
- eine Untereinheit (b), bestehend aus SEQ ID NO: 6,
wobei Untereinheit (b) mit dem carboxylterminalen Ende von Untereinheit (a) verknüpft ist oder Untereinheit (b) in Untereinheit (a) eingefügt ist.

2. Chimärer virusähnlicher Partikel nach Anspruch 1, wobei Untereinheit (b) mit dem carboxylterminalen Ende von Untereinheit (a) durch ein Linker-Polypeptid von bis zu 10 Aminosäuren, welche sich zwischen den Aminosäuresequenzen von Untereinheiten (a) und (b) befinden, verknüpft ist.

3. Chimärer virusähnlicher Partikel nach einem der Ansprüche 1 oder 2, wobei SEQ ID NO: 6 mit R₄₅₂ von SEQ ID NO: 4 verknüpft ist.

4. Chimärer virusähnlicher Partikel nach Anspruch 3, wobei das Fusionsprotein eine Aminosäuresequenz aufweist, wie in SEQ ID NO: 7 festgelegt ist.

5. Chimärer virusähnlicher Partikel nach Anspruch 1, **dadurch gekennzeichnet, dass** das Fusionsprotein zusätzlich ein oder zwei Linker-Polypeptide von bis zu 15 Aminosäuren, welche sich zwischen der Aminosäuresequenz von Untereinheit (b) und der Aminosäuresequenz von Untereinheit (a) befindet, umfasst, wobei Untereinheit (b) in Untereinheit (a) eingefügt ist.

6. Chimärer virusähnlicher Partikel nach einem der Ansprüche 1 oder 5, wobei SEQ ID NO: 6 zwischen den Aminosäuren L₄₃₆ und K₄₃₇ von SEQ ID NO: 4 eingefügt ist.

7. Chimärer virusähnlicher Partikel nach Anspruch 6, wobei das Fusionsprotein eine Aminosäuresequenz aufweist, wie in SEQ ID NO: 8 festgelegt ist.

8. Chimärer virusähnlicher Partikel nach einem der Ansprüche 1 oder 5, wobei SEQ ID NO: 6 zwischen den Aminosäuren A₄₄₁ und F₄₄₂ von SEQ ID NO: 4 eingefügt ist.

9. Chimärer virusähnlicher Partikel nach Anspruch 8, wobei das Fusionsprotein eine Aminosäuresequenz aufweist, wie in SEQ ID NO: 9 festgelegt ist.

10. Chimärer virusähnlicher Partikel nach einem der Ansprüche 1 oder 5, wobei SEQ ID NO: 6 zwischen den Aminosäuren A₄₅₀ und I₄₅₁ von SEQ ID NO: 4 eingefügt ist.

11. Chimärer virusähnlicher Partikel nach Anspruch 10, wobei das Fusionsprotein eine Aminosäuresequenz aufweist, wie in SEQ ID NO: 10 festgelegt ist.

12. Verfahren zur Gewinnung von chimären virusähnlichen Partikeln nach einem der Ansprüche 1 bis 11, welches das Kultivieren einer Wirtszelle umfasst, die eine Nukleinsäure, die ein Fusionsprotein nach einem der Ansprüche 1 bis 11 unter Bedingungen, welche die Expression des Fusionsproteins ermöglichen, codiert und die Zusammenfügung des Fusionsproteins zum Bilden chimärer virusähnlicher Partikel umfasst.

13. Verfahren zur Gewinnung chimärer virusähnlicher Partikel nach dem vorhergehenden Anspruch, wobei es zusätzlich das Isolieren oder Reinigen der chimären virusähnlichen Partikel umfasst.

14. Verwendung des chimären virusähnlichen Partikels nach einem der Ansprüche1 bis 11 zur Herstellung eines Arzneimittels.

15. Verwendung des virusähnlichen Partikels nach einem der Ansprüche 1 bis 11 zur Herstellung eines Arzneimittels zur Vorbeugung und/oder Behandlung einer durch das humane Papillomavirus verursachten Neoplasie.

16. Verwendung des virusähnlichen Partikels nach einem der Ansprüche 1 bis 11 zur Herstellung eines Arzneimittels zur Vorbeugung und/oder Behandlung eines Zervixkarzinoms.

17. Pharmazeutische Zusammensetzung, welche den chimären virusähnlichen Partikel nach einem der Ansprüche 1 bis 11 umfasst.

18. Pharmazeutische Zusamensetzung nach Anspruch 17, welche zusätzlich einen pharmazeutisch annehmbaren Träger umfasst.

## Revendications

1. Particule pseudo-virale chimérique (PPV) formée par une protéine de fusion comprenant :
- une sous-unité (a) constituée du fragment de protéine de IBDV, VP2₄₅₂, avec ID de SÉQ. N° : 4, et
- une sous-unité (b) constituée de l'ID de SÉQ. N° : 6.
dans laquelle la sous-unité (b) est liée à l'extrémité carboxy-terminale de la sous-unité (a) ou la sous-unité (b) est insérée dans la sous-unité (a).

2. Particule pseudo-virale chimérique, selon la revendication 1, dans laquelle la sous-unité (b) est liée à l'extrémité carboxy-terminale de la sous-unité (a) au moyen d'un polypeptide lieur allant jusqu'à 10 acides aminés situés entre les séquences d'acides aminés des sous-unités (a) et (b).

3. Particule pseudo-virale chimérique, selon l'une quelconque des revendications 1 ou 2, dans laquelle l'ID de SÉQ. N° : 6 est lié à R₄₅₂ avec l'ID de SÉQ. N° : 4.

4. Particule pseudo-virale chimérique, selon la revendication 3, dans laquelle la protéine de fusion a une séquence d'acides aminés telle que présentée dans l'ID de SÉQ. N° : 7.

5. Particule pseudo-virale chimérique, selon la revendication 1, **caractérisée en ce que** la sous-unité (b) insérée dans la sous-unité (a) comprend en outre un ou deux polypeptides lieurs de jusqu'à 15 acides aminés situés entre la séquence d'acides aminés de la sous-unité (b) et la séquence d'acides aminés de la sous-unité (a).

6. Particule pseudo-virale chimérique, selon l'une quelconque des revendications 1 ou 5, dans laquelle l'ID de SÉQ. N° : 6 est inséré entre les acides aminés L₄₃₆ et K₄₃₇ avec l'ID de SÉQ. N° : 4.

7. Particule pseudo-virale chimérique, selon la revendication 6, dans laquelle la protéine de fusion a une séquence d'acides aminés telle que présentée dans l'ID de SÉQ. N° : 8.

8. Particule pseudo-virale chimérique, selon l'une quelconque des revendications 1 ou 5, dans laquelle l'ID de SÉQ. N° : 6 est inséré entre les acides aminés A₄₄₁ et F₄₄₂ avec l'ID de SÉQ. N° : 4.

9. Particule pseudo-virale chimérique, selon la revendication 8, dans laquelle la protéine de fusion a une séquence d'acides aminés telle que présentée dans l'ID de SÉQ. N° : 9.

10. Particule pseudo-virale chimérique, selon l'une quelconque des revendications 1 ou 5, dans laquelle l'ID de SÉQ. N° : 6 est inséré entre les acides aminés A₄₅₀ et I₄₅₁ avec l'ID de SÉQ. N° : 4.

11. Particule pseudo-virale chimérique, selon la revendication 10, dans laquelle la protéine de fusion a une séquence d'acides aminés telle que présentée dans l'ID de SÉQ. N° : 10.

12. Procédé d'obtention de particules pseudo-virales chimériques, selon l'une quelconque des revendications 1 à 11, qui inclut la culture d'une cellule hôte comprenant un acide nucléique codant pour une protéine de fusion, selon l'une quelconque des revendications 1 à 11, dans des conditions permettant l'expression de ladite protéine de fusion et l'assemblage de ladite protéine de fusion pour former des particules pseudo-virales chimériques.

13. Procédé d'obtention de particules pseudo-virales chimériques, selon la revendication précédente, et comprenant en outre l'isolement ou la purification des dites particules pseudo-virales chimériques.

14. Utilisation de la particule pseudo-virale chimérique, selon l'une quelconque des revendications 1 à 11 pour la préparation d'un médicament.

15. Utilisation de la particule pseudo-virale, selon l'une quelconque des revendications 1 à 11 pour la préparation d'un médicament pour la prévention et/ou le traitement d'une néoplasie causée par le virus du papillome humain.

16. Utilisation de la particule pseudo-virale, selon l'une quelconque des revendications 1 à 11 pour la préparation d'un médicament pour la prévention et/ou le traitement du cancer du col de l'utérus.

17. Composition pharmaceutique comprenant la particule pseudo-virale chimérique, selon l'une quelconque des revendications 1 à 11.

18. Composition pharmaceutique, selon la revendication 17, comprenant en outre un véhicule pharmaceutiquement acceptable.


### SEQUENCE LISTING

<110> CHIMERA PHARMA S.L.
<120> VACCINES FOR THE TREATMENT OF NEOPLASIAS BASED ON VIRAL CAPSIDS OF BIRNAVIRUS CONTAINING HUMAN PAPILLOMAVIRUS ANTIGENS
<130> EP2214.2
<150> P200930967
   <151> 2009-11-06
<160> 21
<170> PatentIn version 3.5
<210> 1
   <211> 512
   <212> PRT
   <213> Artificial Sequence
<220>
   <223> pVP2 of IBDV Soroa strain
<400> 1
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg Ile Ala Val Pro Val Val Ser Thr Leu Phe Pro
       450                 455                 460
   Pro Ala Ala Pro Leu Ala His Ala Ile Gly Glu Gly Val Asp Tyr Leu
   465                 470                 475                 480
   Leu Gly Asp Glu Ala Gln Ala Ala Ser Gly Thr Ala Arg Ala Ala Ser
                   485                 490                 495
   Gly Lys Ala Arg Ala Ala Ser Gly Arg Ile Arg Gln Leu Thr Leu Ala
               500                 505                 510
<210> 2
   <211> 98
   <212> PRT
   <213> Artificial Sequence
<220>
   <223> E7 of VPH-16
<400> 2
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro
<210> 3
   <211> 63
   <212> PRT
   <213> Artificial Sequence
<220>
   <223> E7 of VPH-16 in which amino acids 1 to 35 have been deleted
<400> 3
   Asp Glu Ile Asp Gly Pro Ala Gly Gln Ala Glu Pro Asp Arg Ala His
   1               5                   10                  15
   Tyr Asn Ile Val Thr Phe Cys Cys Lys Cys Asp Ser Thr Leu Arg Leu
               20                  25                  30
   Cys Val Gln Ser Thr His Val Asp Ile Arg Thr Leu Glu Asp Leu Leu
           35                  40                  45
   Met Gly Thr Leu Gly Ile Val Cys Pro Ile Cys Ser Gln Lys Pro
       50                  55                  60
<210> 4
   <211> 452
   <212> PRT
   <213> Artificial Sequence
<220>
   <223> pVP2 of IBDV Soroa strain truncated in its carboxy-terminal end at position 452
<400> 4
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg
       450
<210> 5
   <211> 518
   <212> PRT
   <213> Artificial Sequence
<220>
   <223> Fusion protein
<400> 5
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg Pro Arg Asp Glu Ile Asp Gly Pro Ala Gly Gln
       450                 455                 460
   Ala Glu Pro Asp Arg Ala His Tyr Asn Ile Val Thr Phe Cys Cys Lys
   465                 470                 475                 480
   Cys Asp Ser Thr Leu Arg Leu Cys Val Gln Ser Thr His Val Asp Ile
                   485                 490                 495
   Arg Thr Leu Glu Asp Leu Leu Met Gly Thr Leu Gly Ile Val Cys Pro
               500                 505                 510
   Ile Cys Ser Gln Lys Pro
           515
<210> 6
   <211> 54
   <212> PRT
   <213> Artificial Sequence
<220>
   <223> E7 of VPH-16 in which amino acids 1 to 44 have been deleted
<400> 6
   Ala Glu Pro Asp Arg Ala His Tyr Asn Ile Val Thr Phe Cys Cys Lys
   1               5                   10                  15
   Cys Asp Ser Thr Leu Arg Leu Cys Val Gln Ser Thr His Val Asp Ile
               20                  25                  30
   Arg Thr Leu Glu Asp Leu Leu Met Gly Thr Leu Gly Ile Val Cys Pro
           35                  40                  45
   Ile Cys Ser Gln Lys Pro
       50
<210> 7
   <211> 509
   <212> PRT
   <213> Artificial Sequence
<220>
   <223> Fusion protein
<400> 7
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg Pro Arg Ala Glu Pro Asp Arg Ala His Tyr Asn
       450                 455                 460
   Ile Val Thr Phe Cys Cys Lys Cys Asp Ser Thr Leu Arg Leu Cys Val
   465                 470                 475                 480
   Gln Ser Thr His Val Asp Ile Arg Thr Leu Glu Asp Leu Leu Met Gly
                   485                 490                 495
   Thr Leu Gly Ile Val Cys Pro Ile Cys Ser Gln Lys Pro
               500                 505
<210> 8
   <211> 525
   <212> PRT
   <213> Artificial Sequence
<220>
   <223> Fusion protein
<400> 8
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Met Arg Pro Gly Gly Gly Gly Ser Ala Glu Pro Asp
   435 440 445
   Arg Ala His Tyr Asn Ile Val Thr Phe Cys Cys Lys Cys Asp Ser Thr
   450 455 460
   Leu Arg Leu Cys Val Gln Ser Thr His Val Asp Ile Arg Thr Leu Glu
   465 470 475 480
   Asp Leu Leu Met Gly Thr Leu Gly Ile Val Cys Pro Ile Cys Ser Gln
   485 490 495
   Lys Pro Gly Gly Gly Gly Ser Thr Ser Gly Pro His Leu Lys Ile Ala
   500 505 510
   Gly Ala Phe Gly Phe Lys Asp Ile Ile Arg Ala Ile Arg
   515 520 525
<210> 9
   <211> 515
   <212> PRT
   <213> Artificial Sequence
<220>
   <223> Fusion protein
<400> 9
   Met Thr Asn Leu Ser Asp Gln Thr Gln Gln Ile Val Pro Phe Ile Arg
   1 5 10 15
   Ser Leu Leu Met Pro Thr Thr Gly Pro Ala Ser Ile Pro Asp Asp Thr
   20 25 30
   Leu Glu Lys His Thr Leu Arg Ser Glu Thr Ser Thr Tyr Asn Leu Thr
   35 40 45
   Val Gly Asp Thr Gly Ser Gly Leu Ile Val Phe Phe Pro Gly Phe Pro
   50 55 60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Leu Arg Pro Ala Glu Pro Asp
           435                 440                 445
   Arg Ala His Tyr Asn Ile Val Thr Phe Cys Cys Lys Cys Asp Ser Thr
       450                 455                 460
   Leu Arg Leu Cys Val Gln Ser Thr His Val Asp Ile Arg Thr Leu Glu
   465                 470                 475                 480
   Asp Leu Leu Met Gly Thr Leu Gly Ile Val Cys Pro Ile Cys Ser Gln
                   485                 490                 495
   Lys Pro Thr Ser Gly Pro Gln Ala Phe Gly Phe Lys Asp Ile Ile Arg
               500                 505                 510
   Ala Ile Arg
           515
<210> 10
   <211> 515
   <212> PRT
   <213> Artificial Sequence
<220>
   <223> Fusion protein
<400> 10
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Met Arg Pro Ala Glu Pro Asp Arg Ala His Tyr Asn Ile Val
       450                 455                 460
   Thr Phe Cys Cys Lys Cys Asp Ser Thr Leu Arg Leu Cys Val Gln Ser
   465                 470                 475                 480
   Thr His Val Asp Ile Arg Thr Leu Glu Asp Leu Leu Met Gly Thr Leu
                   485                 490                 495
   Gly Ile Val Cys Pro Ile Cys Ser Gln Lys Pro Thr Ser Gly Pro Gln
               500                 505                 510
   Ala Ile Arg
           515
<210> 11
   <211> 516
   <212> PRT
   <213> Artificial Sequence
<220>
   <223> Fusion protein
<400> 11
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Arg Pro Arg Asp Glu Ile Asp Gly Pro Ala Gly Gln Ala Glu
       450                 455                 460
   Pro Asp Arg Ala His Tyr Asn Ile Val Thr Phe Cys Cys Lys Cys Asp
   465                 470                 475                 480
   Ser Thr Leu Arg Leu Cys Val Gln Ser Thr His Val Asp Ile Arg Thr
                   485                 490                 495
   Leu Glu Asp Leu Leu Met Gly Thr Leu Gly Ile Val Cys Pro Ile Cys
               500                 505                 510
   Ser Gln Lys Pro
           515
<210> 12
   <211> 515
   <212> PRT
   <213> Artificial Sequence
<220>
   <223> Fusion protein
<400> 12
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Lys Ile Ala Gly Ala Phe Gly Phe Lys Asp Ile Ile
   435 440 445
   Arg Arg Pro Arg Asp Glu Ile Asp Gly Pro Ala Gly Gln Ala Glu Pro
   450 455 460
   Asp Arg Ala His Tyr Asn Ile Val Thr Phe Cys Cys Lys Cys Asp Ser
   465 470 475 480
   Thr Leu Arg Leu Cys Val Gln Ser Thr His Val Asp Ile Arg Thr Leu
   485 490 495
   Glu Asp Leu Leu Met Gly Thr Leu Gly Ile Val Cys Pro Ile Cys Ser
   500 505 510
   Gln Lys Pro
   515
<210> 13
   <211> 512
   <212> PRT
   <213> Artificial Sequence
<220>
   <223> Fusion protein
<400> 13
   Met Thr Asn Leu Ser Asp Gln Thr Gln Gln Ile Val Pro Phe Ile Arg
   1 5 10 15
   Ser Leu Leu Met Pro Thr Thr Gly Pro Ala Ser Ile Pro Asp Asp Thr
   20 25 30
   Leu Glu Lys His Thr Leu Arg Ser Glu Thr Ser Thr Tyr Asn Leu Thr
   35 40 45
   Val Gly Asp Thr Gly Ser Gly Leu Ile Val Phe Phe Pro Gly Phe Pro
   50 55 60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
   325 330 335
   Ile His Gly Gly Asn Tyr Pro Gly Ala Leu Arg Pro Val Thr Leu Val
   340 345 350
   Ala Tyr Glu Arg Val Ala Thr Gly Ser Val Val Thr Val Ala Gly Val
   355 360 365
   Ser Asn Phe Glu Leu Ile Pro Asn Pro Glu Leu Ala Lys Asn Leu Val
   370 375 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Lys Ile Ala Gly Ala Phe Gly Phe Lys Asp Arg Pro
   435 440 445
   Arg Asp Glu Ile Asp Gly Pro Ala Gly Gln Ala Glu Pro Asp Arg Ala
   450 455 460
   His Tyr Asn Ile Val Thr Phe Cys Cys Lys Cys Asp Ser Thr Leu Arg
   465 470 475 480
   Leu Cys Val Gln Ser Thr His Val Asp Ile Arg Thr Leu Glu Asp Leu
   485 490 495
   Leu Met Gly Thr Leu Gly Ile Val Cys Pro Ile Cys Ser Gln Lys Pro
   500 505 510
<210> 14
   <211> 509
   <212> PRT
   <213> Artificial Sequence
<220>
   <223> Fusion protein
<400> 14
   Met Thr Asn Leu Ser Asp Gln Thr Gln Gln Ile Val Pro Phe Ile Arg
   1 5 10 15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Arg Pro Arg Asp Glu
           435                 440                 445
   Ile Asp Gly Pro Ala Gly Gln Ala Glu Pro Asp Arg Ala His Tyr Asn
       450                 455                 460
   Ile Val Thr Phe Cys Cys Lys Cys Asp Ser Thr Leu Arg Leu Cys Val
   465                 470                 475                 480
   Gln Ser Thr His Val Asp Ile Arg Thr Leu Glu Asp Leu Leu Met Gly
                   485                 490                 495
   Thr Leu Gly Ile Val Cys Pro Ile Cys Ser Gln Lys Pro
               500                 505
<210> 15
   <211> 507
   <212> PRT
   <213> Artificial Sequence
<220>
   <223> Fusion protein
<400> 15
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Arg Pro Arg Asp Glu Ile Asp
           435                 440                 445
   Gly Pro Ala Gly Gln Ala Glu Pro Asp Arg Ala His Tyr Asn Ile Val
       450                 455                 460
   Thr Phe Cys Cys Lys Cys Asp Ser Thr Leu Arg Leu Cys Val Gln Ser
   465 470 475 480
   Thr His Val Asp Ile Arg Thr Leu Glu Asp Leu Leu Met Gly Thr Leu
   485 490 495
   Gly Ile Val Cys Pro Ile Cys Ser Gln Lys Pro
   500 505
<210> 16
   <211> 513
   <212> PRT
   <213> Artificial Sequence
<220>
   <223> Fusion protein
<400> 16
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
   180 185 190
   Met Val Ala Thr Cys Asp Ser Ser Asp Arg Pro Arg Val Tyr Thr Ile
   195 200 205
   Thr Ala Ala Asp Asp Tyr Gln Phe Ser Ser Gln Tyr Gln Pro Gly Gly
   210 215 220
   Val Thr Ile Thr Leu Phe Ser Ala Asn Ile Asp Ala Ile Thr Ser Leu
   225 230 235 240
   Ser Val Gly Gly Glu Leu Val Phe Arg Thr Ser Val His Gly Leu Val
   245 250 255
   Leu Gly Ala Thr Ile Tyr Leu Ile Gly Phe Asp Gly Thr Thr Val Ile
   260 265 270
   Thr Arg Ala Val Ala Ala Asn Asn Gly Leu Thr Thr Gly Thr Asp Asn
   275 280 285
   Leu Met Pro Phe Asn Leu Val Ile Pro Thr Asn Glu Ile Thr Gln Pro
   290 295 300
   Ile Thr Ser Ile Lys Leu Glu Ile Val Thr Ser Lys Ser Gly Gly Gln
   305 310 315 320
   Ala Gly Asp Gln Met Ser Trp Ser Ala Arg Gly Ser Leu Ala Val Thr
   325 330 335
   Ile His Gly Gly Asn Tyr Pro Gly Ala Leu Arg Pro Val Thr Leu Val
   340 345 350
   Ala Tyr Glu Arg Val Ala Thr Gly Ser Val Val Thr Val Ala Gly Val
   355 360 365
   Ser Asn Phe Glu Leu Ile Pro Asn Pro Glu Leu Ala Lys Asn Leu Val
   370 375 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Lys Ile Ala Gly Ala Phe Gly Phe Lys Asp Ile Ile
   435 440 445
   Arg Ala Ile Arg Arg Pro Arg Pro Ala Gly Gln Ala Glu Pro Asp Arg
   450 455 460
   Ala His Tyr Asn Ile Val Thr Phe Cys Cys Lys Cys Asp Ser Thr Leu
   465 470 475 480
   Arg Leu Cys Val Gln Ser Thr His Val Asp Ile Arg Thr Leu Glu Asp
   485 490 495
   Leu Leu Met Gly Thr Leu Gly Ile Val Cys Pro Ile Cys Ser Gln Lys
   500 505 510
   Pro
<210> 17
   <211> 512
   <212> PRT
   <213> Artificial Sequence
<220>
   <223> Fusion protein
<400> 17
   Met Thr Asn Leu Ser Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Arg Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
   355 360 365
   Ser Asn Phe Glu Leu Ile Pro Asn Pro Glu Leu Ala Lys Asn Leu Val
   370 375 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Lys Ile Ala Gly Ala Phe Gly Phe Lys Asp Ile Ile
   435 440 445
   Arg Ala Ile Arg Arg Pro Arg Ala Gly Gln Ala Glu Pro Asp Arg Ala
   450 455 460
   His Tyr Asn Ile Val Thr Phe Cys Cys Lys Cys Asp Ser Thr Leu Arg
   465 470 475 480
   Leu Cys Val Gln Ser Thr His Val Asp Ile Arg Thr Leu Glu Asp Leu
   485 490 495
   Leu Met Gly Thr Leu Gly Ile Val Cys Pro Ile Cys Ser Gln Lys Pro
   500 505 510
<210> 18
   <211> 1151
   <212> DNA
   <213> Artificial Sequence
<220>
   <223> DNA sequence of the cloning insert containing the kanamycin resistant gene and unique restrictions sites for NotI and SpeI and the unique restriction site for Bsp 120I.
<400> 18
   gactgcggcc gccctatcgt caattattac ctccacgggg agagcctgag caaactggcc      60
   tcaggcattt gagaagcaca cggtcacact gcttccggta gtcaataaac cggtaaacca     120
   gcaatagaca taagcggcta tttaacgacc ctgccctgaa ccgacgaccg ggtcgaattt     180
   gctttcgaat ttctgccatt catccgctta ttatcactta ttcaggcgta gcaaccaggc     240
   gtttaagggc accaataact gccttaaaaa aattacgccc cgccctgcca ctcatcgcag     300
   tactgttgta attcattaag cattctgccg acatggaagc catcacaaac ggcatgatga     360
   acctgaatcg ccagcggcat cagcaccttg tcgccttgcg tataatattt gcccatggtg     420
   aaaacggggg cgaagaagtt gtccatattg gccacgttta aatcaaaact ggtgaaactc     480
   acccagggat tggctgagac gaaaaacata ttctcaataa accctttagg gaaataggcc     540
   aggttttcac cgtaacacgc cacatcttgc gaatatatgt gtagaaactg ccggaaatcg     600
   tcgtggtatt cactccagag cgatgaaaac gtttcagttt gctcatggaa aacggtgtaa     660
   caagggtgaa cactatccca tatcaccagc tcaccgtctt tcattgccat acgtaattcc     720
   ggatgagcat tcatcaggcg ggcaagaatg tgaataaagg ccggataaaa cttgtgctta     780
   tttttcttta cggtctttaa aaaggccgta atatccagct gaacggtctg gttataggta     840
   cattgagcaa ctgactgaaa tgcctcaaaa tgttctttac gatgccattg ggatatatca     900
   acggtggtat atccagtgat ttttttctcc attttagctt ccttagctcc tgaaaatctc     960
   gacaactcaa aaaatacgcc cggtagtgat cttatttcat tatggtgaaa gttggaacct    1020
   cttacgtgcc gatcaacgtc tcattttcgc caaaagttgg cccagggctt cccggtatca    1080
   acagggacac caggatttat ttattctgcg aagtgatctt ccgtcacagg tatttactag    1140
   tgggcccagt c                                                         1151
<210> 19
   <211> 180
   <212> DNA
   <213> Artificial Sequence
<220>
   <223> DNA sequences of the instert encoding E7 of VPH-16 in which amino acids
   1 to 44 have been deleted, and which presents in its ends unique restrictions sites for Bsp 120I and SpeI
<400> 19
   ccagggcccg ctgaaccaga tagagcccat tacaacattg tcacattctg ttgtaaatgt      60
   gattcaacat tgagattatg tgttcaatct acccatgttg atattagaac attagaagat     120
   ttgttaatgg gtacattagg tattgtttgt ccaatttgct cacaaaaacc tactagtggt     180
<210> 20
   <211> 211
   <212> DNA
   <213> Artificial Sequence
<220>
   <223> DNA sequences of the instert encoding E7 of VPH-16 in which amino acids
   1 to 44 have been deleted, and the spacer polypeptide, and which presents
   in its ends unique restriction sites for Bsp 120I and SpeI.
<400> 20
   ccagggcccg gtggtggagg ttcagctgaa ccagatagag cccattacaa cattgtcaca      60
   ttctgttgta aatgtgattc aacattgaga ttatgtgttc aatctaccca tgttgatatt     120
   agaacattag aagatttgtt aatgggtaca ttaggtattg tttgtccaat ttgctcacaa     180
   aaacctggag gaggtggatc aactagtggt c                                    211
<210> 21
   <211> 5
   <212> PRT
   <213> Artificial Sequence
<220>
   <223> Spacer sequence
<400> 21
   Gly Gly Gly Gly Ser
   1               5